# EUROPEAN PATENT APPLICATION

(11) **EP 2 561 841 A1**
(43) Date of publication of application: **27.02.2013**
(21) Application number: 11768956.2
(22) Date of filing: 15.04.2011
(51) Int. Cl.: A61F 5/11

(54) **INGROWN NAIL CORRECTION TOOL, METHOD FOR MANUFACTURING INGROWN NAIL CORRECTION TOOL, AND METHOD FOR IMPROVING DURABILITY AND CORRECTIVE FORCE OF INGROWN NAIL CORRECTION TOOL**

(30) Priority: 16.04.2010 JP 2010095260
(71) Applicant: Furukawa Techno Material Co., Ltd, Kanagawa 254-0016 (JP); Furukawa Electric Co., Ltd., Chiyoda-ku Tokyo 100-8322 (JP); Ishida, Kiyohito, Miyagi 980-0011 (JP)
(72) Inventor: ISHIDA, Kiyohito, Sendai-shi Miyagi 980-0011 (JP); TANAKA, Toyonobu, Hiratsuka-shi Kanagawa 254-0016 (JP); HABU, Tetsushi, Hiratsuka-shi Kanagawa 254-0016 (JP); HAGIWARA, Takuzo, Tokyo 100-8322 (JP)
(74) Representative: Strehl Schübel-Hopf & Partner
(86) International application number: PCT/JP2011/059382
(87) International publication number: WO 2011/129435

(57) **Abstract**

The longitudinal direction (rolling direction) of a cold processed plate material 11 is indicated by the letter L and the width direction by the letter W. The layout direction of an ingrown nail correction tool material 13 is arranged such that the longitudinal direction of a material 13a of a body portion (corresponding to correction tool body 3) substantially corresponds to the width direction W, and the direction in which a nail holder portion material 13b (corresponding to nail holder portion 5) of a nail holder portion is formed substantially corresponds to a direction in which the cold processed plate material is processed. That is to say, the material 13 of the ingrown nail correction tool is integrally cut out from the cold processed plate material 11 and processed such that the longitudinal direction of the correction tool body 3 of an ingrown nail correction tool 1 is substantially perpendicular (direction W) to the direction in which the cold processed plate material 11 is processed; and the direction in which the nail holder portion 5 is formed substantially corresponds to the direction in which the cold processed plate material 11 is processed (direction L).

## Description

### TECHNICAL FIELD OF THE INVENTION

The present invention relates to ingrown nail correction tools and the like with excellent durability that are installed on a toenail of a human body to correct an ingrown nail having an ingrown portion at a side portion of the nail.

### BACKGROUND ART

There are cases in which, for example, a curved nail, which is the condition where a toenail is curved, and an ingrown nail, which is the condition where a side portion of a toenail is curved to grow into the flesh around the side portion of the nail, (hereinafter, these conditions are collectively referred to as ingrown nail) occur. The ingrown nail is the condition where the nail is curved to cause a side portion of the nail to dig into the flesh and the nail grows into the tissue.

For ingrown nails, there is, for example, an ingrown nail correction tool that a support body having a plurality of linear correction effectors of super-elastic alloy is applied onto a surface of an ingrown nail to correct a curve of the ingrown nail to a normal state by a recovery force of the correction effectors (Patent Document 1).

Further, there is a deformed nail correction tool which is manufactured using as a material an elastic metal strip cut out such that a direction in which a cold processed material is processed is the longitudinal direction and in which a plurality of hooked claws engaged with a free edge of the nail are provided at front edge portions on the free edge of the nail side such that a direction of processing that is the longitudinal direction of the metal strip is the nail width direction (Patent Document 2).

### PRIOR ART DOCUMENTS

### PATENT DOCUMENTS

Patent Document 1: Japanese Patent Publication No. H09-253111A
Patent Document 2: Japanese Patent Publication No. 2007-244852A

### SUMMARY OF THE INVENTION

### TECHNICAL PROBLEM

In the ingrown nail correction tool described in Patent Document 1, the plurality of linear correction effectors are formed in parallel. This makes the production steps complicated. Furthermore, since spaces are formed between the correction effectors, there arises a problem that a corrective force is dispersed. If the correction effectors are formed in close contact with each other, it is necessary to use a large number of correction effectors. This increases the costs. Moreover, each correction effector must be bonded with a fabric tape when installed on an ingrown nail. Thus, it is not possible to install in a single operation.

On the contrary, the problems of Patent Document 1 do not arise with the deformed nail correction tool of Patent Document 2, because the plate-like shape elastic metal strip is used so that the coercive force dose not disperse and, furthermore, since it is not necessary to use a plurality of components, the correction tool can be installed in a single operation.

Meanwhile, users of the ingrown nail correction tool and the like described above make their daily lives with the ingrown nail correction tool and the like being installed on an ingrown nail. That is to say, users put on socks and the like and walk for daily lives with the correction tool being installed. Accordingly, deformation stress associated with the movement of toes during walking and the like is applied to the correction tool. Further, installation of the correction tool is continued until the ingrown nail is completely corrected, meaning that the correction tool is used continuously for several weeks to several months. The period of use varies according to severity of the ingrown nail of the person wearing the tool, and the correction tool is required to have sufficient durability for a long-term use. A substantially constant corrective force is effected even after the correction has progressed, or the corrective force is decreased only slightly as the correction has progressed. That is to say, the ingrown nail correction tool is used under an environment in which repeated deformation stress is applied for a long period of time.

For example, when a user wears the deformed nail correction tool of Patent Document 2 on his/her toe and walks, stress is concentrated on the hooked claw portions, which are installation portions by which the correction tool is installed on the nail, whereby the greatest deformation stress is applied to the hooked claw portions. Therefore, it is desired that the deformed nail correction tool firmly hold the nail without breakage of the nail holding even when repeated stress is applied as described above. However, no attention has been focused on durability against such repeated stress associated with walking. Hence, there has been no such a material that has been developed to improve durability of an installation portion.

In view of use under the above-described environment in which repeated deformation stress is applied, the present invention is aimed at providing ingrown nail correction tools and the like that are excellent in durability of especially an installation portion (nail holder portion). The present invention is also aimed at making it possible to improve not only durability of an ingrown nail application tool but also a corrective force of a correction tool.

### TECHNICAL SOLUTION

In order to achieve the above aim, the first invention provides an ingrown nail correction tool made of a super-elastic alloy for correcting an ingrown nail, comprising: a plate-like shape correction tool body portion; and a plurality of nail holder portions formed at side portions of the correction tool body portion, wherein the ingrown nail correction tool is made of a material prepared by subjecting a cold processed plate material of a Cu-Al-Mn based super-elastic alloy to shape-memory heat treatment, and is formed by being folded such that a longitudinal direction of the correction tool body portion is substantially perpendicular to a direction in which the cold processed plate material is processed and that a direction in which the nail holder portions are formed substantially corresponds to the direction in which the cold processed plate material is processed, and the nail holder portions are capable of holding a front edge of an ingrown nail that is a subject of correction with the longitudinal direction of the correction tool body portion being a width direction of the ingrown nail. The shape-memory heat treatment (memory heat treatment) refers to heat treatment for imparting shape-memory characteristics or super-elastic characteristics to a subject super-elastic alloy, but in the present invention, it refers to heat treatment for imparting super-elastic characteristics.
The present invention is characterized in that it is excellent in durability and can improve a corrective force.
Further, the Cu-Al-Mn based super-elastic alloy, which is a material of the correction tool for an ingrown nail, may be an alloy comprising 3 to 10 wt% of Al, 5 to 20 wt% of Mn, Cu as the balance and an inevitable impurity. The Cu-Al-Mn based super-elastic alloy, which is a material of the correction tool for an ingrown nail, may be an alloy comprising 3 to 10 wt% of Al, 5 to 20 wt% of Mn, 0.001 to 10 wt% of one or more ofNi, Co, Fe, Ti, V, Cr, Si, Nb, Mo, W, Sn, Sb, Mg, P, Be, Zr, Zn, B, C, Ag and misch metal, Cu as the balance and an inevitable impurity. By use of a material of the alloy composition defined above, an ingrown nail correction tool with high durability can reliably be obtained.

It is preferable that the nail holder portions be formed by folding along a first folding part near a front end portion a tongue-shaped strip formed substantially perpendicularly to the longitudinal direction of the correction tool body portion, and also folding the tongue-shaped strip along a second folding part near a boundary portion with the correction tool body portion; at the second folding part, the tongue-shaped strip is folded at an angle of 180° or larger with respect to a direction in which the tongue-shaped strip is formed; and at the first folding part, the tongue-shaped strip is folded at an angle larger than 180° with respect to the nail holder portions between the first folding part and the second folding part.

The correction tool body portion may be curved in a planar direction along a curving direction of an ingrown nail that is substantially perpendicular to the direction in which the nail holder portions are formed. Further, the ingrown nail correction tool may be surface treated to improve corrosion resistance. The first invention makes it possible to obtain an ingrown nail correction tool with excellent durability which maintains installation properties with respect to an ingrown nail even when it is installed on an ingrown nail and used for a long time. The tool obtained is also adequate in terms of anti-stress corrosion cracking properties. Further, in cases of severe ingrown nails, the body portion of the ingrown nail correction tool is curved in the planar direction of the curving direction of the ingrown nail to facilitate application of the ingrown nail correction tool for ease of use. Further, by surface treating the ingrown nail correction tool to improve corrosion resistance, the corrosion resistance of the ingrown nail correction tool can be improved.

Further, the folding angle at the second folding part of the nail holder portion is set to 180° or larger to obtain a greater nail holding force. Further, although stress applied to the nail holder portions increases as a result of increasing the holding force of the nail holder portions, the holding force is less likely to be deteriorated by repeated load because of high durability. That is to say, an ingrown nail correction tool is obtained that is durable for a long time under use conditions.

The second invention provides a method of manufacturing an ingrown nail correction tool for correcting an ingrown nail that is made of a super-elastic alloy and comprises a plate-like shape correction tool body portion and a plurality of nail holder portions formed at side portions of the correction tool body portion, the method comprising: forming by cold rolling a cold processed plate material which is a material of a Cu-Al-Mn based super-elastic alloy; integrally cutting out an ingrown nail correction tool material such that the longitudinal direction of the correction tool body portion is substantially perpendicular to the direction in which the cold processed plate material is processed and that the direction in which the tongue-shaped strip substantially perpendicular to the longitudinal direction of the correction tool body portion substantially corresponds to the direction in which the cold processed plate material is processed; folding at a first folding part near a front end portion the tongue-shaped strip at an angle larger than 180° with respect to the direction in which the tongue-shaped strip is formed and folding at a second folding part near a boundary portion with the correction tool body portion the tongue-shaped strip at an angle of 180° or larger with respect to the tongue-shaped strip between the first folding part and the second folding part to thereby form the nail holder portions; and subjecting the ingrown nail correction tool in this state to shape-memory heat treatment.

The third invention provides a method of improving durability and a correction force of an ingrown nail correction tool for correcting an ingrown nail that is made of a super-elastic alloy and comprises a plate-like shape correction tool body portion and a plurality of nail holder portions formed at side portions of the correction tool body portion, the method comprising: forming by cold rolling a cold processed plate material which is a material of a Cu-Al-Mn based super-elastic alloy; integrally cutting out an ingrown nail correction tool material such that the longitudinal direction of the correction tool body portion is substantially perpendicular to the direction in which the cold processed plate material is processed and that the direction in which the tongue-shaped strip substantially perpendicular to the longitudinal direction of the correction tool body portion substantially corresponds to the direction in which the cold processed plate material is processed; folding at a first folding part near a front end portion the tongue-shaped strip at an angle larger than 180° with respect to the direction in which the tongue-shaped strip is formed and folding at a second folding part near a boundary portion with the correction tool body portion the tongue-shaped strip at an angle of 180° or larger with respect to the tongue-shaped strip between the first folding part and the second folding part to thereby form the nail holder portions; and subjecting the ingrown nail correction tool in this state to shape-memory heat treatment.

The second and third inventions make it possible to obtain an ingrown nail correction tool having excellent durability even under a long-time use conditions.

### ADVANTAGEOUS EFFECT OF THE INVENTION

The present invention can provide ingrown nail correction tools and the like having excellent durability with consideration for use under an environment in which deformation stress is applied repeatedly.

### BRIEF DESCRIPTION OF THE DRAWINGS

[Figure 1] A perspective view showing an ingrown nail correction tool 1
[Figure 2] A rear view showing the ingrown nail correction tool 1
[Figure 3] A cross-sectional view along the line A-A indicated in Figure 2
[Figure 4] A figure showing the ingrown nail correction tool 1 being installed
[Figure 5] A cross-sectional view along the line B-B indicated in Figure 4,
showing the ingrown nail correction tool 1 being installed.
[Figure 6] A figure showing a layout direction of an ingrown nail correction tool material 13 in a cold processed plate 11
[Figure 7] A figure showing a method of bending process of the ingrown nail correction tool 1
[Figure 8] A figure showing stress-strain curves for the direction of processing
[Figure 9] A figure showing a method of testing for bend durability the ingrown nail correction tool 1
[Figure 10] A figure showing a method of testing for environmental durability of the ingrown nail correction tool 1
[Figure 11] A figure showing ingrown nail correction tools 1a and 1b.

### EMBODIMENTS

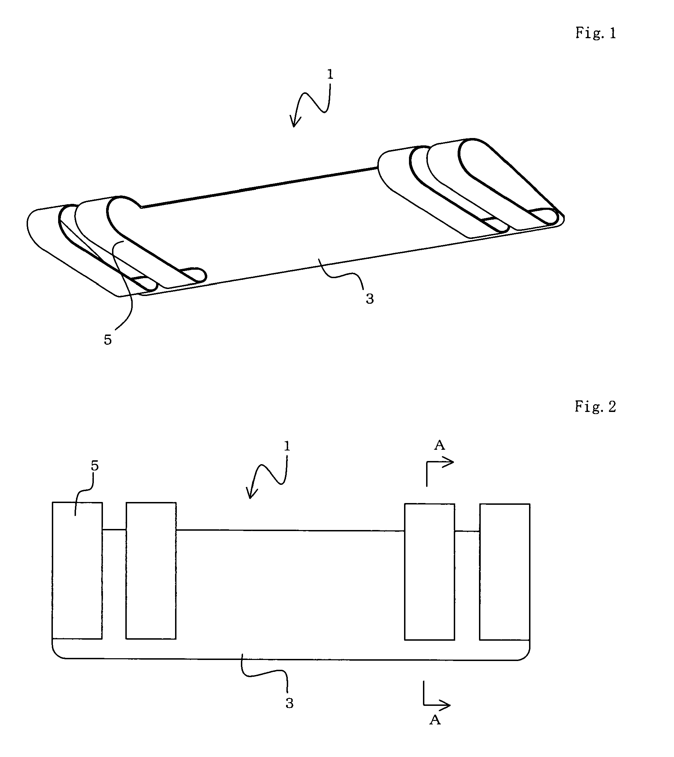

Embodiments of the present invention will be described below with reference to the drawings. Figures 1 and 2 show an ingrown nail correction tool 1; Figure 1 shows a rear perspective view, and Figure 2 shows a rear view. The ingrown nail correction tool 1 mainly includes a correction tool body 3, which is in the shape of a flat plate, and a plurality of nail holder portions 5 formed at side portions (side portions in longitudinal direction) of the correction tool body 3.

The correction tool body 3 is a substantially-rectangular plate-like portion. The longitudinal direction of the correction tool body 3 corresponds to the width direction of an ingrown nail. The length of the correction tool body 3 in the longitudinal direction is set as appropriate to the size of a nail on which the tool is to be installed; for example, about 12 to 20 mm. The width of the correction tool body 3 is about 3 to 5 mm, **in consideration of** recovery force, wearability and the like. The shape of the correction tool body 3 is usually a completely flat plate, but in some cases the shape may be changed such that the correction direction and corrective force can be adjusted in advance as appropriate to the shape of an ingrown nail. For example, in cases of severe ingrown portions, formation of a curved, bending portion may facilitate installation.

The nail holder portions 5 are formed on one side portion of the correction tool body 3 along the longitudinal direction. The nail holder portions 5 are formed at multiple parts of the correction tool body 3 including, for example, parts near both end portions of the correction tool body 3 in the longitudinal direction.

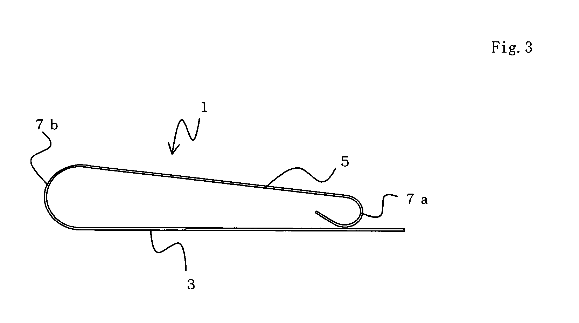

Figure 3 is a cross-sectional view along the line A-A indicated in Figure 2, showing the shape of the nail holder portion 5. The nail holder portion 5 is folded along a folding part 7b on one side of the correction tool body 3 and is further folded along a folding part 7a in the same direction. Details of folding angles of the nail holder portion 5 and the like will be described below.

In the ingrown nail correction tool 1, the correction tool body 3 and the nail holder portions 5 are integrally formed. The ingrown nail correction tool 1 is made of a super-elastic alloy and has super-elastic characteristics under conditions in which it is used by a user. A preferred material of the ingrown nail correction tool 1 is a Cu-Al-Mn based super-elastic alloy. This alloy may be a ternary alloy containing 3 to 10 wt% of Al, 5 to 20 wt% of Mn and Cu as the balance (including inevitable elements) and, as necessary, the alloy may further contain 0.001 to 10 wt% of one or more of Ni, Co, Fe, Ti, V, Cr, Si, Nb, Mo, W, Sn, Sb, Mg, P, Be, Zr, Zn, B, C, Ag and misch metal.

As to conditions for obtaining the super-elasticity effect, it is necessary that the Cu-Al-Mn based alloy turn into the β single phase at high temperature (two phase texture of β+α at low temperature) in the phase diagram of the Cu-Al-Mn based alloy. With less than 3 wt% of Al, the β phase is not formed. With more than 10 wt% of Al, the alloy is likely to embrittle. Hence, Al is preferably 3 to 10 wt%.

Further, Mn stabilizes the β phase especially at low temperature and improves cold processability. With less than 5 wt% of Mn, The foregoing effects are not sufficiently produced. On the other hand, excessive addition of more than 20 wt% is likely to adversely affect the super-elasticity effect. Hence, Mn is preferably 5 to 20 wt%.

As to other additive elements, Ni, Co, Fe, Sn, Sb and Be have an effect of reinforcing a matrix. Ti fixes N and O to render them harmless. W, V, Nb, Mo and Zr improve hardness and wear resistance. Mg improves hot processability and toughness. P and misch metal are added as a deoxidizing agent to improve toughness. Zn increases the shape memory temperature. B and C reinforce the grain boundary to improve processability and toughness. Ag improves cold processability. Accordingly, at least one of the foregoing elements is added within the range of 0.001 to 10 wt% so that properties such as, mainly, processability, hardness, wear resistance and toughness can be improved, and as to the amounts of addition, no problem will occur as long as the amounts are within the above-specified ranges.

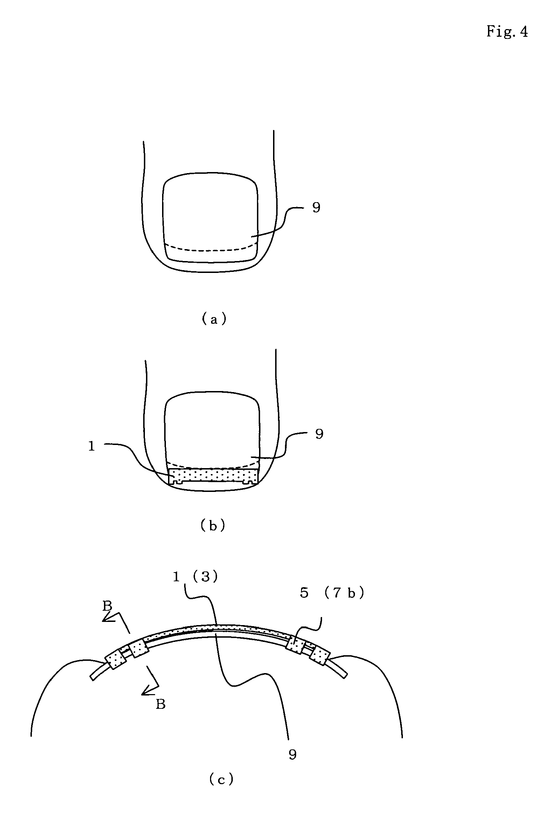

Next, a method of using the ingrown nail correction tool 1 will be described. Figure 4 shows a state of the ingrown nail correction tool 1 being used. Figure 4(a) is a plan view showing an ingrown nail 9. Figure 4(b) is a plan view showing the ingrown nail 9 on which the ingrown nail correction tool 1 is installed. Figure 4(c) is an enlarged front view showing the ingrown nail 9 on which the ingrown nail correction tool 1 is installed.

On the ingrown nail 9 having both side portions grown in the flesh as shown in Figure 4(a), the ingrown nail correction tool 1 is installed as shown in Figure 4(b). Here, the longitudinal direction of the correction tool body corresponds to the width direction of the ingrown nail 9. The ingrown nail correction tool 1 is placed on an upper surface of the ingrown nail 9, and the nail holder portions 5 are installed on a front edge portion of the ingrown nail 9. In other words, a free edge of the nail is inserted into the nail holder portions 5.

As shown in Figure 4(c), both side portions of the ingrown nail 9 are curved significantly to be embedded in the flesh. Thus, the correction tool body 3 of the ingrown nail correction tool 1 is deformed along the shape of the ingrown nail 9. Since the correction tool body 3 has super-elastic characteristics at use temperature, it tends to recover the shape it had before the installation (e.g., shape of flat plate). Further, the ingrown nail correction tool 1 holds the ingrown nail 9 at portions near both end portions of the ingrown nail correction tool 1. Thus, the shape of the ingrown nail 9 is corrected by the recovery force of the ingrown nail correction tool 1 to the shape of the correction tool body 3 that it had before the installation.

The ingrown nail correction tool 1 is used continuously while being installed. The period needed for the correction varies depending on the shape of the ingrown nail 9 and other conditions; in some cases, several weeks to several months may be needed. In other words, during that period, the user makes his/her daily life with the ingrown nail correction tool 1 being installed.

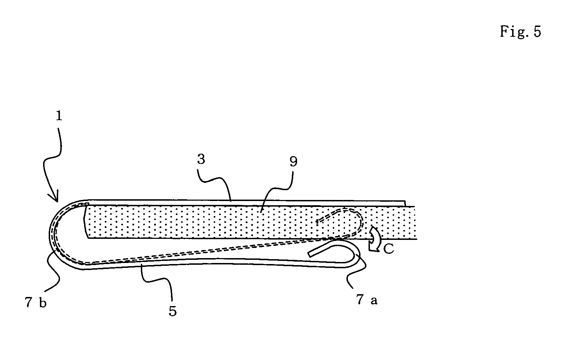

Figure 5 is a cross-sectional view along the line B-B indicated in Figure 4. As described above, the front edge portion of the nail holder portion 5 is folded along the folding part 7a, which is the first folding part, and a portion near a boundary portion between the nail holder portion 5 and the correction tool body 3 is folded along the folding part 7b, which is the second folding part.

With the correction tool body 3 being placed on an upper surface of the ingrown nail 9, the front edge of the nail holder portion 5 is inserted under the ingrown nail 9 from the free edge of the ingrown nail 9. In other words, the free edge of the nail is inserted between the correction tool body 3 and the nail holder portion 5.

To install the ingrown nail correction tool 1 on the free edge of the nail firmly, a nail holding force of the nail holder portion 5 is important. Specifically, when the free edge of the nail is inserted, the nail holder portion 5 is elastically deformed in the direction in which a space is opened between the correction tool body 3 and the nail holder portion 5 (direction of arrow C in the figure) in response to the insertion of the free edge of the nail, thereby holding the free edge of the nail. Hence, in order to obtain an even greater holding force, it is preferable to apply greater deformation to the ingrown nail 9 at the time of the insertion into the free edge of the nail.

As described above, the ingrown nail correction tool 1 is used continuously for a long time by the user until the correction of the ingrown nail is completed. Further, since the user makes his/her daily life with the correction tool being installed, when, for example, the user walks, deformation stress is also applied to the ingrown nail correction tool 1 following the movement of toes and changes in the shape of shoes and the like that are associated with the walking. The portions at which the greatest stress is likely to be concentrated so that the greatest deformation stress is applied at that time are especially the folding parts 7a and 7b of the nail holder portion 5. Hence, in order to obtain higher durability of the ingrown nail correction tool 1, it is necessary to obtain durability in the folding directions of the folding parts 7a and 7b of the nail holder portion 5. A method of processing to obtain such durability will be described below.

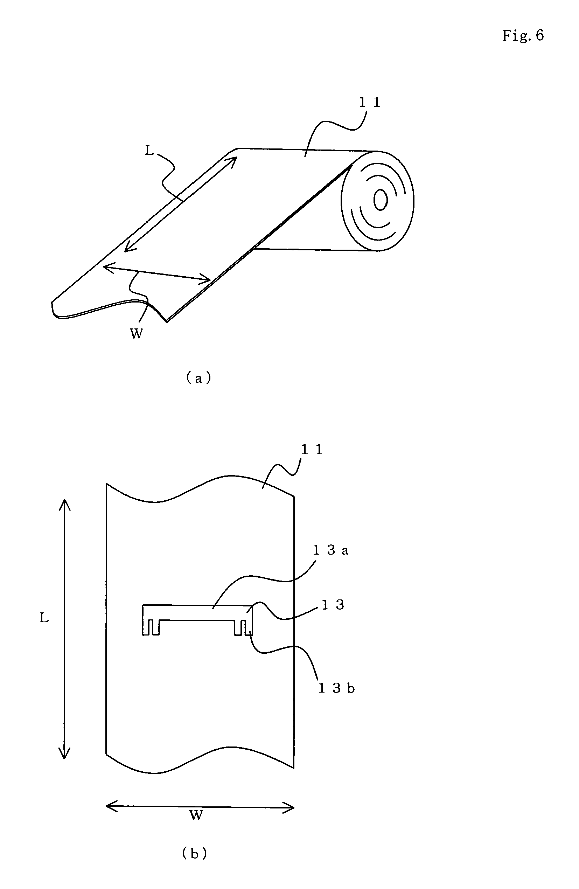

Figure 6 shows a cold processed plate material 11, which is a material of the ingrown nail correction tool 1. The cold processed plate material 11 is manufactured as follows. First, a metal adjusted to have predetermined components is melted and cast into an ingot. A surface of the ingot thus obtained is shaved to remove external oxides and the like. For example, the surface is shaved by about 2.5 mm.

Then, hot forging is conducted at 700°C to 900°C, and hot rolling at 500°C to 700°C. Thereafter, the resulting hot rolled plate is subjected to annealing and then cold rolling for several times. The rolling rate of one cold rolling can be set within the range of 60% or lower in both the cold rolling conducted immediately after the hot rolling and the cold rolling conducted after process annealing. The upper limit of the rolling rate after the annealing is set to 60% because when the rolling is conducted at a rolling rate higher than the above range, both end portions of the rolled material will generate cracks, that is to say, so-called edge crack will occur, causing the yield of the plate material to decrease. Moreover, rolling properties will be deteriorated due to work hardening. In the present invention, for example, the annealing is conducted immediately after the hot rolling and then the process annealing is conducted repeatedly during the cold rolling; in both cases, the annealing and the cold rolling are conducted repeatedly with the rolling rate after annealing being set to about 40% to obtain a cold processed plate material 11. The process annealing is conducted normally at 400 to 700°C, because the annealing effect will not be produced if the temperature is lower than 400°C, and the effect will be saturated if the temperature exceeds 700°C. Hence, substantially sufficient annealing effect will be obtained if the annealing is conducted within the above range. As to the annealing time of the process annealing, the process annealing is conducted normally within the range of 1 to 60 minutes. In the present invention, the process annealing was conducted at 600°C for 10 minutes.

Then, the cold processed plate is subjected to slitter processing to obtain stripes with a necessary width, and the stripes are pressed into ingrown nail correction tools. The slitter width is set as appropriate to the width of a final product and the like. Specifically, for example, the slitter width is set to a value calculated by adding trimming margins to the integral multiple of the width of the final product, but may be set to a size calculated by adding trimming margins of both sides to the width of the final product. After punching with a press and forming processing, shape-memory heat treatment is conducted. The shape-memory heat treatment is conducted within the range of 800 to 950°C and the range of 1 to 30 minutes, followed by aging treatment conducted within the range of 100 to 200°C and the range of 10 to 60 minutes. This is because sufficient super-elasticity effect cannot be obtained outside the range of 800 to 950°C. Especially when the shape-memory heat treatment temperature exceeds 950°C, a part of a crystal grain boundary of the material may be locally melted. The aging treatment is conducted within the range of 100 to 200°C and the range of 10 to 60 minutes so that the transformation temperature can be controlled as appropriate. Beyond the above ranges, the transformation temperature cannot be controlled as appropriate. In the shape-memory heat treatment conducted in the present invention, shape-memory heat treatment (water cooling) is conducted in the β single phase range, e.g., 900°C for 10 minutes, followed by aging treatment (air cooling) at 200°C for 15 minutes. At the end, surface treatment (e.g., barrel polishing, pickling and, as necessary, coating) is conducted, whereby a final product is completed.

As shown in Figure 6(a), the longitudinal direction (rolling direction) of the cold processed sheet material 11 in the steps described above is denoted by L and the width direction by W. Here, the layout direction (the cutting out direction) nail correction tool material 13 is determined such that the longitudinal direction of a body portion material 13a (corresponding to correction tool body 3) substantially corresponds to the width direction (direction W) and the direction in which a nail holder portion material 13b (corresponding to nail holder portion 5) substantially corresponds to the direction in which the cold processed plate material is processed (direction L). That is to say, the ingrown nail correction tool material 13 is integrally cut out from the cold processed plate material 11 and processed such that the longitudinal direction of the correction tool body 3 of the ingrown nail correction tool 1 is substantially perpendicular (direction W) to the direction in which the cold processed plate material 11 is processed and the direction in which the nail holder portion 5 is formed substantially corresponds to the direction in which the cold processed plate material 11 is processed (direction L).

The direction of cutting out the ingrown nail correction tool material 13 is defined as described above for the following reasons. In general, cold processed plate materials are likely to exhibit anisotropy in the rolling direction and in the width direction. This is because there is a correlation between the crystal direction of the metal structure and processability (slip direction). Further, these materials exhibit different behaviors in all material properties that an ingrown nail correction tool is required to have, such as material strength, residual strain and repeated bending, depending on the relation with the rolling direction. Therefore, anisotropy of material properties of the plate material must be considered according to an intended use and, furthermore, there have been almost no case in which repeated bending characteristics are studied. Accordingly, in cases of cutting out a product material from the foregoing cold processed plate material, it is necessary to consider the relation of material properties of the product, such as material strength, residual strain and repeated bending characteristics, with anisotropy in the direction in which the plate material is processed. Repeated bending characteristics relate to the breakage lifetime of an installation portion (nail holder portion) of an ingrown nail correction tool and, furthermore, material strength is an important factor that relates to a corrective force of the product. Hence, it is the most important object in the product development of ingrown nail correction tools to determine a balance of the above described properties. Directional properties of the material properties described above will be described below.

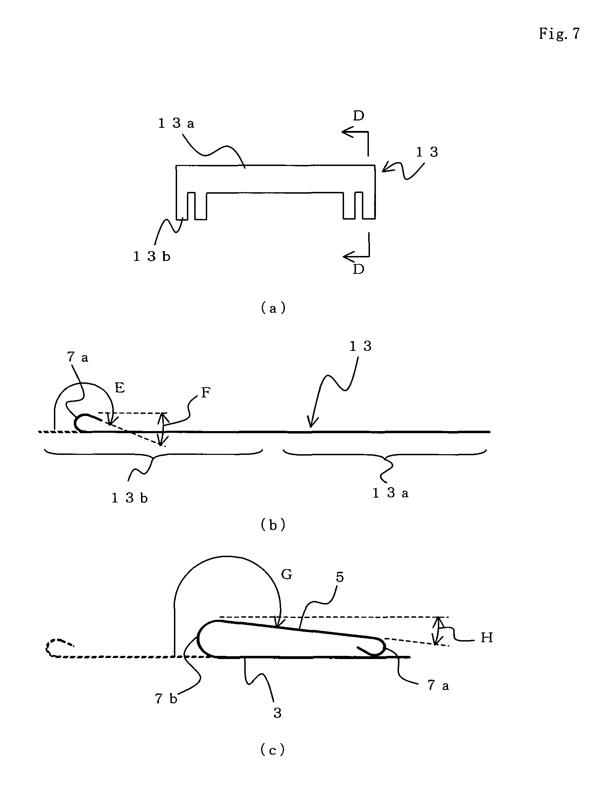

Figure 7 shows the steps of total working process of the ingrown nail correction tool material 13 that has been cut out. From the step of cutting out to the step of final bending processing, the material is processed by a single press machine using a plurality of molds. As shown in Figure 7(a), the ingrown nail correction tool material 13 includes the body portion material 13a and the nail holder portion material 13b formed at side portions of the body portion material 13a in the longitudinal direction. In other words, a plurality of nail holder portion materials 13b, which are tongue-shaped strips, are formed in the direction substantially perpendicular to the longitudinal direction of the body portion material 13a, which is in the shape of a plate.

Figures 7(b) and 7(c) are cross-sectional views along the line D-D indicated in Figure 7(a), showing the steps of processing the nail holder portion material 13b. First, as shown in Figure 7(b), a portion near a front edge portion of the nail holder portion material 13b is folded along the folding part 7a (direction of arrow E in the figure). At this time, the folding angle of the folding part 7a preferably exceeds 180°. In other words, it is preferable to fold at an angle that is larger by the angle F than a folding angle at which surfaces of the nail holder portion material that are formed with the folding part 7a being their boundary are parallel to each other (i.e., 180° bending). The angle F is preferably larger than 0° and not larger than about 30°. That is to say, the folding angle of the folding part 7a is preferably larger than 180° and not larger than about 210°, because bending at an angle larger than 210° merely increases the degree of processing but the bending effect stays about the same.

Next, as shown in Figure 7(c), a portion near the boundary portion between the nail holder portion material 13b and the body portion material 13a is folded along the folding part 7b (direction of arrow G in the figure). The folding part 7b is folded in the same direction as the folding part 7a. Here, the folding angle of the folding part 7b is preferably 180° or larger. Furthermore, it is preferable to fold at an angle that is larger by the angle H than a folding angle at which the nail holder portion material 13b and the body portion material 13a are parallel to each other (i.e., 180° bending) with the folding part 7b being their boundary. The angle H is about 0 to 30°, preferably about 5 to 25°. That is to say, the folding angle of the folding part 7b is preferably about 185 to 205°. The folding angle is set to exceed 180°, for example 185° or larger, to thereby increase the nail holding force of the nail holder portions. Processing exceeding 205° is difficult because the degree of processing increases. Thus the folding angle of the folding part 7b is preferably about 185 to 205°.

The nail holder portion 5 is formed as described above. It is to be noted that the direction in which the nail holder portion 5 is formed is the direction in which the nail holder portion material 13b is formed in Figure 7(a). The nail holder portion 5 is formed by folding along the folding parts 7a and 7b and each folding angle is set as described above so that the front edge portion of the nail holder portion 5 would not be caught on a nail when being inserted into the nail and when being removed from the nail. Further, since the aperture width of the portion in which a nail is to be inserted is decreased and, therefore, greater deformation is applied when a nail is inserted, the nail can be held by a greater holding force. This makes it possible to prevent the correction tool from shifting at the time of installation and the like.

The shape of the nail holder portion is designed as described above to increase the nail holding force; as a result, more deformation stress is applied to the ingrown nail correction tool 1 (nail holder portion 5) at the time of installation. Further, as described above, the nail holder portion 5 is a portion to which the greatest repeated stress is applied as a result of following the movement of toes associated with the user's walking and the like when the ingrown nail correction tool is being installed. Hence, as a result of obtaining the strong holding force described above, the ingrown nail correction tool 1 (nail holder portion 5) is used under more repeated stress.

The ingrown nail correction tool of the present invention is designed such that the direction in which the greatest repeated stress is applied, that is to say, the direction in which the nail holder portion is formed is the direction in which the cold processed plate material having the highest durability of the repeated bending is processed. Therefore, the amount of deformation at the time of insertion of the nail holder portion into a nail is increased to thereby obtain high durability even under long-term use under more stress.

### EXAMPLES

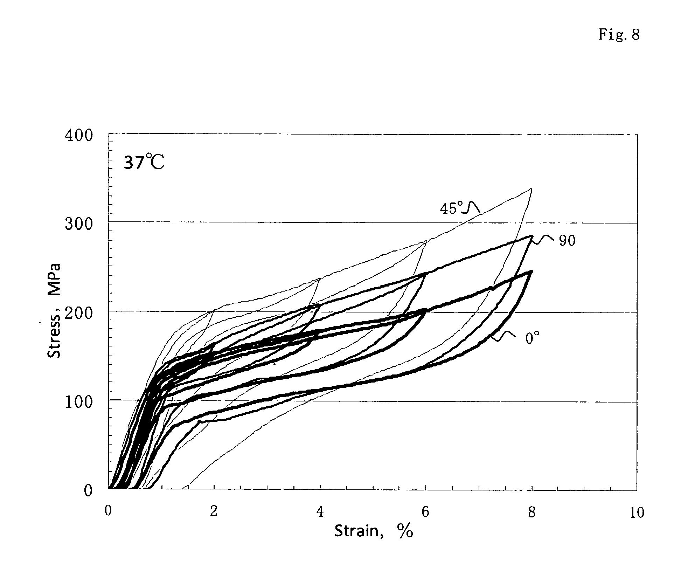

Stress-strain characteristics were studied for 0° (same as direction of processing), 90° (perpendicular to direction of processing) and 45° (between 0° and 90°) with respect to the direction of cold-processing. Figure 8 shows stress-strain curves for the respective angles with respect to the direction (L) in which a cold processed plate material is processed in the present invention. In the figure, 0° shows an evaluation of a test specimen cut out along the direction of cold processing with respect to the direction of cutting-out (direction of processing), and the angle 90° shows an evaluation of a test specimen cut out along the direction (direction W) substantially perpendicular to the direction of cold processing with respect to the direction of cutting-out (width direction). Further, 45° shows an evaluation of a test specimen of a diagonal direction in-between.

The material used contained 8.1 wt% Al, 10 wt% Mn and substantially Cu as the balance. Each component was analyzed in accordance with JISH1055 and JISH1057. As to conditions for the manufacture of the cold rolled plate material, first, a metal that had been adjusted to have predetermined components was melted to cast an 85ϕ×0.3m ingot, and the resulting ingot was shaved to a diameter of about 80ϕ.

Then, hot forging was conducted at 700°C to 900°C to form two forged materials with a thickness of 15 mm, a width of 50 mm and a length of 1 m from the ingot. Thereafter, the forged materials were subjected to hot rolling at 500°C to 700°C to form a hot rolled plate with a thickness of about 2 mm, a width of about 60 mm and a length of about 6 m. Then, the hot rolled plate thus obtained was subjected repeatedly to cold rolling and process annealing (at 600°C for 10 minutes) at a processing rate of 40%. The resulting cold rolled plate had a thickness of about 0.2 mm, a width of about 65 mm and a length of about 50 m. Thereafter, as shape-memory heat treatment, the cold rolled plate was subjected to solution treatment (at 900°C for 5 minutes) and thereafter aging treatment (at 150°C for 20 minutes).

### (Tensile test)

Test specimens for tensile test were cut out in respective directions from the cold rolled plate material thus obtained. The test specimens were prepared to have a thickness of 0.2 mm, a width of 10 mm and a length of about 100 mm. The test specimens thus prepared were subjected to shape-memory heat treatment and thereafter a tensile test. The tensile test was conducted by holding the test specimen with chucks such that the distance between the chucks was 50 mm, and then pulling at a speed of 2 mm/min. It is to be noted that material properties largely depend on temperature; in view of use conditions of the ingrown nail correction tool of the present invention, properties at 37°C will be described in the following description.

As shown in Figure 8, mechanical properties of the material vary depending on the layout direction of the test specimen with respect to the direction in which the cold rolled plate material was processed. Table 1 shows stress and residual strain for the respective directions that are read from Figure 8. Considering the feature how the ingrown nail correction tool is used, the use range is likely to be at a strain of 4% or lower. Thus, the following description will be based on 2 to 4% strain.

**[Table 1]**

| Layout direction | Stress (MPa) | | | | Residual strain (%) | | | |
|---|---|---|---|---|---|---|---|---|
| | 2% | 4% | 6% | 8% | After 2% | After 4% | After 6% | After 8% |
| 0° | 154 | 179 | 203 | 246 | 0.13 | 0.18 | 0.26 | 0.49 |
| 45° | 202 | 238 | 280 | 339 | 0.21 | 0.35 | 0.66 | 1.39 |
| 90° | 164 | 208 | 244 | 286 | 0.20 | 0.32 | 0.49 | 0.72 |

As shown in Table 1, the layout direction of 45° has the largest stress, but the residual strain is large. Furthermore, as to 45°, production is difficult in view of material yield and production steps. As to 90° (perpendicular to direction of processing), the stress and residual strain are slightly greater than those of 0° (corresponding to direction of processing). However, the residual strain (after 4% strain) is 0.4% or smaller in both directions and, furthermore, a difference from the test result of 0° is only about 0.14% (after 4% strain); the difference is little. Therefore, there is no significant difference in stress-strain characteristics between 0° and 90°. Further, the residual strain (after 8% strain) of the direction of 45° exceeds 1%; the residual strain is large. On the other hand, the residual strains of the directions of 0° and 90° are only less than 1%.

Since the stress of the direction of 90° is higher than the stress of the direction of 0°, a greater corrective force than that of the material of the direction of 0° with the same thickness can be obtained by, for example, designing the direction of 90° as the longitudinal direction of the correction tool body as in the present invention. Further, the plate thickness can be made smaller than that of a material of the same corrective force to thereby improve user comfort. Further, as to the direction of 45°, although the corrective force can be increased, since the residual strain is high, the direction of 45° is not considered as a suitable material for the present intended use in terms of residual strain.

### (Repeated bending test)

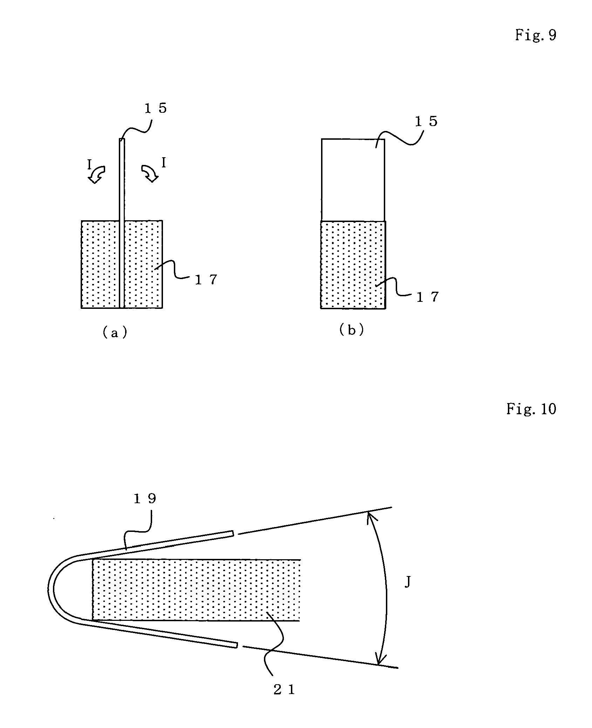

Next, repeated bending tests were conducted to confirm durability against repeated bending of the folding parts of the nail holder portion. The repeated bending tests (bend durability test) were conducted for test specimens of the respective layout directions of 0°, 45° and 90°. Figure 9 shows a method of testing bend durability. The test specimens were cut out from the cold rolled plate material of the above-described conditions (final thickness: 0.2 mm) such that each direction corresponded to the longitudinal direction. The test specimens with a length of 30 mm and a width of 10 mm were cut out from the cold rolled plate material. The test specimens thus prepared were subjected to shape-memory heat treatment and thereafter repeated bending tests.

As shown in Figures 9(a) and 9(b), a test specimen 15 was sandwiched between a pair of jigs 17. The jigs 17 were made of steel and completely sandwiched the test specimen 15 substantially up to a central part. To the test specimen 15 in this state, repeated bending deformation of 90° was applied along the planar direction of the test specimen 15 (direction of jigs 17). The test specimen temperature was set to 37°C in view of use conditions. The repeated bending tests were conducted with a tension of 50MPa (upper side of drawing), which was lower than a bearing force, being applied to the test specimen 15. The results are shown in Table 2.

**[Table 2]**

| Layout Direction | Number of breakage (n=5) | | | | | |
|---|---|---|---|---|---|---|
| | No. 1 | No.2 | No.3 | No.4 | No.5 | Average |
| 0° | 56 | 71 | 63 | 66 | 61 | 64 |
| 45° | 36 | 27 | 38 | 34 | 36 | 32 |
| 90° | 25 | 30 | 28 | 30 | 32 | 28 |

The repeated bending tests were conducted five times for each direction. As to the number of bending, bending in one direction was counted as once, and one reciprocating bending in both directions (right and left directions in Figure 9(a)) was counted as twice. The number of breakage is the number of bending conducted until a breakage in the bent portion occurred.

From Table 2 it is understood that the test specimen of the cutting-out direction of 0° had bend durability that was more than about twice the test specimens of the other directions. In other words, it is understood that the direction that substantially corresponded to the direction in which the cold rolled plate material was processed had the best bend durability and was excellent in durability. Hence, the highest durability can be obtained by making the nail holder portion correspond to the direction in accordance with the cold rolled direction of the rolled plate material processed as in the present invention.

### (Stress corrosion test)

Then, in view of use conditions, stress corrosion tests were conducted using artificial sweat. As to the test solution of the stress corrosion tests, a test solution for corrosion resistance test defined under JISB7285 and prepared by dissolving 50 g of lactic acid and 100 g of sodium chloride in 11 of water was used. Absorbent cotton was placed in a sealed vessel, and the test solution was poured to completely moisten the absorbent cotton. The test specimens to which stress had been applied were placed on the upper side of the absorbent cotton via a jig and exposed in a test solution atmosphere. The test temperature was set to about 38°C.

Figure 10 shows a method of applying stress to the test specimens for the stress corrosion tests. The test specimens were cut out from the cold rolled plate material of the above-described conditions (final thickness: 0.2 mm) such that the longitudinal direction of each test specimen corresponded to each layout direction. The test specimens that had been bent substantially parallel (180°) in a horseshoe shape to have an aperture width of 1 mm were subjected to shape-memory heat treatment, and thereafter stress corrosion tests were conducted. A jig 21 in the shape of a plate with a thickness of 1.5 mm, which was slightly thicker than the aperture width (1 mm), was inserted from the aperture of the test specimen 19 to forcibly open the test specimen 19. The aperture angle (J in the figure) was set to 25°. While the test specimen 19 was opened by the jig 21, the jig 21 was held such that only the jig 21 was in contact with the test solution, and after 2 weeks, an occurrence of stress corrosion cracking was visually evaluated. As a result, no stress corrosion cracking was found in any layout direction, and no difference based on the layout direction was found. Corrosion resistance can be improved by subjecting the ingrown nail correction tool to surface treatment such as resin coating for improving corrosion resistance such as polyester. This makes it possible to prevent discoloration of a surface of the ingrown nail correction tool material caused by corrosion when the ingrown nail correction tool is installed. At the same time, various pigments may be mixed with the resin used to thereby obtain a highly-fashionable ingrown nail correction tool.

As described above, in the ingrown nail correction tool of the present invention, the correction tool body portion that applies a corrective force to an ingrown nail is in the 90° direction, which is substantially perpendicular to the rolling direction. This makes it possible to set the corrective force of the ingrown nail correction tool high and the residual strain low. Further, since the nail holder portion to which the greatest stress is applied substantially corresponds to the direction in which the cold rolled plate material is processed and has the best durability, high durability can be obtained under a long-term use under repeated stress without any breakage resulting from repeated bending. This makes it possible to avoid troubles such as breakage of the ingrown nail correction tool while the ingrown nail correction tool is being installed.

Accordingly, the nail holding force of the nail holder portion can be increased to thereby improve durability of the ingrown nail correction tool. This effect of improving durability was also confirmed by the user wearing the ingrown nail correction tool.

Particular embodiments of the present invention have been described with reference to the attached drawings, but the technical scope of the present invention is not restricted by the embodiments described above. It should be apparent to those skilled in the art that many more changes and modifications besides those already described are possible without departing from the inventive concepts defined in the appended claims, and it is to be understood that such changes and modifications are naturally encompassed within the scope of the invention.

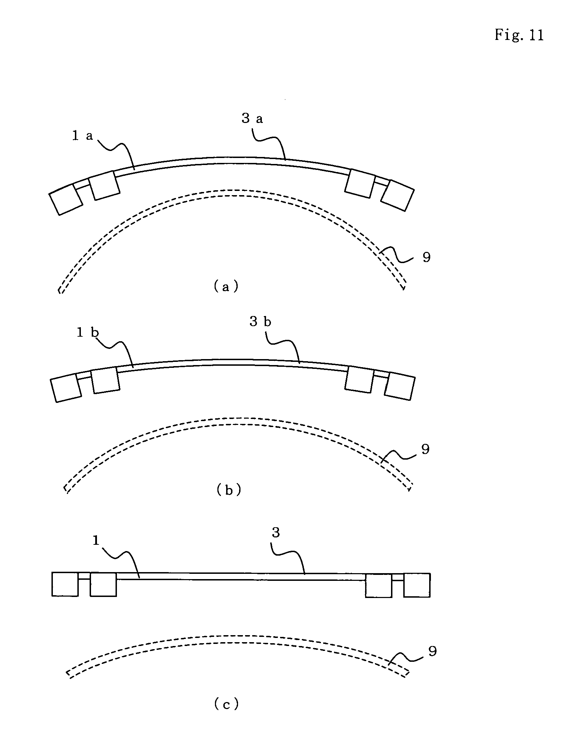

For example, although the ingrown nail correction tool 1 having the correction tool body 3 that is completely flat and in the shape of a flat plate is described as an example, the present invention is not limited to this example. For example, as shown in Figure 11, the correction tool body may be formed to curve in a direction substantially perpendicular to the direction in which the nail holder portion is formed (in longitudinal direction (planar direction) of correction tool body).

Figures 11(a) and (b) are front views showing ingrown nail correction tools 1a and 1b having correction tool bodies 3a and 3b curved at a predetermined curvature in the direction in which nail holder portions are bent. The correction tool bodies 3a and 3b are curved along the deformation direction of the ingrown nail 9 on which the tool is to be installed. That is to say, the correction tool bodies 3a and 3b are shaped to have downward surfaces that are to be applied on a nail, end portions at which nail holding portions are formed, and central portions curved upward. The curvatures of the correction tool bodies 3a and 3b are set as appropriate to the extent of deformation (curvature) of the ingrown nail 9 on which the tools are to be applied.

In such cases, especially in a case in which the ingrown nail 9 is deformed significantly, since the corrective force created by the correction tool is increased, it is necessary to reduce the corrective force to alleviate strain applied to the user wearing the tool. Thus, for example at the beginning of correction, provided that the curvature of the ingrown nail 9 is constant, the ingrown nail correction tool 1a having the correction tool body 3a curved at a curvature slightly smaller than the curvature of the ingrown nail is installed on the ingrown nail 9 for use (Figure 11 (a)).

As the correction of the ingrown nail 9 is progressed, the ingrown nail correction tool 1b which has a curvature slightly smaller than the curvature of the ingrown nail 9 being corrected and is curved at a curvature smaller than that of the ingrown nail correction tool 1a is installed (Figure 11 (b)) in place of the ingrown nail correction tool 1a. Finally, the ingrown nail correction tool 1 having the flat correction tool body 3 that has no curved portion as illustrated by the examples is installed. By this way, the ingrown nail 9 can be corrected step by step.

As described above, it is common to use the ingrown nail correction tool that has a flat correction tool body having no curve. By use of a curved ingrown nail correction tool, a corrective force appropriate for the shape of an ingrown nail can be obtained. This makes it possible to, for example, make an adjustment to reduce discomfort and pain that the user experiences when a strong corrective force is applied at the beginning of the correction. It is to be noted that it is not necessary to use different ingrown nail correction tools having the curved portion in multiple steps as described above. Further, the ingrown nail correction tool having the curved portion may be used until the correction is completed. That is to say, it is possible to move from Figure 11 (a) to Figure 11 (c), or the ingrown nail correction tool 1b shown in Figure 11 (b) may be used from the beginning to the end.

### EXPLANATION OF REFERENCE NUMERALS

- 1, 1a, 1b: Ingrown nail correction tool
- 3, 3a, 3b: Correction tool body
- 5: Nail holder portion
- 7a, 7b: Folding part
- 9: Ingrown nail
- 11: Cold processed plate material
- 13: Ingrown nail correction tool material
- 13a: Body portion material
- 13b: Nail holder portion material
- 15: Test specimen
- 17: Jig
- 19: Test specimen
- 21: Jig

## Claims

1. An ingrown nail correction tool made of a super-elastic alloy for correcting an ingrown nail, comprising:
a plate-like shape correction tool body portion; and
a plurality of nail holder portions formed at side portions of the correction tool body portion,
wherein the ingrown nail correction tool is made of a material prepared by subjecting a cold processed plate material of a Cu-Al-Mn based super-elastic alloy to shape-memory heat treatment, and is formed by being folded such that a longitudinal direction of the correction tool body portion is substantially perpendicular to a direction in which the cold processed plate material is processed and that a direction in which the nail holder portions are formed substantially corresponds to the direction in which the cold processed plate material is processed, and the nail holder portions are capable of holding a front edge of an ingrown nail that is a subject of correction with the longitudinal direction of the correction tool body portion being a width direction of the ingrown nail.

2. The ingrown nail correction tool of Claim 1 having excellent durability and being capable of improving a corrective force.

3. The ingrown nail correction tool of Claim 1 or 2, wherein the Cu-Al-Mn based super-elastic alloy which is a material of the ingrown nail correction tool is an alloy comprising 3 to 10 wt% of Al, 5 to 20 wt% of Mn, Cu as the balance and an inevitable impurity.

4. The ingrown nail correction tool of Claim 1 or 2, wherein the Cu-Al-Mn based super-elastic alloy which is a material of the ingrown nail correction tool is an alloy comprising 3 to 10 wt% of Al, 5 to 20 wt% of Mn, 0.001 to 10 wt% of one or more of Ni, Co, Fe, Ti, V, Cr, Si, Nb, Mo, W, Sn, Sb, Mg, P, Be, Zr, Zn, B, C, Ag, and misch metal, Cu as the balance and an inevitable impurity.

5. The ingrown nail correction tool of Claim 1, wherein:
the nail holder portions are formed by folding along a first folding part near a front end portion a tongue-shaped strip formed substantially perpendicularly to the longitudinal direction of the correction tool body portion, and also folding the tongue-shaped strip along a second folding part near a boundary portion with the correction tool body portion;
at the second folding part, the tongue-shaped strip is folded at an angle of 180° or larger with respect to a direction in which the tongue-shaped strip is formed; and
at the first folding part, the tongue-shaped strip is folded at an angle larger than 180° with respect to the nail holder portions between the first folding part and the second folding part.

6. The ingrown nail correction tool of Claim 1, wherein the correction tool body portion is curved in a planar direction of the correction tool body portion that is substantially perpendicular to the direction in which the nail holder portions are formed.

7. The ingrown nail correction tool of Claim 1, wherein the ingrown nail correction tool is surface treated to improve corrosion resistance.

8. A method of manufacturing an ingrown nail correction tool for correcting an ingrown nail that is made of a super-elastic alloy and comprises a plate-like shape correction tool body portion and a plurality of nail holder portions formed at side portions of the correction tool body portion, the method comprising:
repeating annealing and cold rolling of a hot rolled material which is a material of a Cu-Al-Mn based super-elastic alloy in this order at an annealing temperature of 400 to 700°C and a cold rolling rate of 60% or lower to form a cold processed plate material;
integrally cutting out an ingrown nail correction tool material such that a longitudinal direction of the correction tool body portion is substantially perpendicular to a direction in which the cold processed plate material is processed and a direction in which a tongue-shaped strip is formed and which is substantially perpendicular to the longitudinal direction of the correction tool body portion substantially corresponds to a direction in which the cold processed plate material is processed; and
forming the nail holder portions by folding the tongue-shaped strip along a first folding part near a front end portion at an angle larger than 180° with respect to the direction in which the tongue-shaped strip is formed and also folding the tongue-shaped strip along a second folding part near a boundary portion with the correction tool body portion at an angle of 180° or larger with respect to the tongue-shaped strip between the first folding part and the second folding part, and in this state subjecting the ingrown nail correction tool to shape-memory heat treatment at 800 to 950°C for 1 to 60 minutes and aging treatment at an aging temperature of 100 to 200°C for 10 to 60 minutes.

9. A method of improving durability and a correction force of an ingrown nail correction tool for correcting an ingrown nail that is made of a super-elastic alloy and comprises a plate-like shape correction tool body portion having and a plurality of nail holder portions formed at side portions of the correction tool body portion, the method comprising:
repeating annealing and cold rolling of a hot rolled material which is a material of a Cu-Al-Mn based super-elastic alloy in this order at an annealing temperature of 400 to 700°C and a cold rolling rate of 60% or lower to form a cold processed plate material;
integrally cutting out an ingrown nail correction tool material such that a longitudinal direction of the correction tool body portion is substantially perpendicular to a direction in which the cold processed plate material is processed and a direction in which a tongue-shaped strip is formed and which is substantially perpendicular to the longitudinal direction of the correction tool body portion substantially corresponds to a direction in which the cold processed plate material is processed; and
forming the nail holder portions by folding the tongue-shaped strip along a first folding part near a front end portion at an angle larger than 180° with respect to the direction in which the tongue-shaped strip is formed and also folding the tongue-shaped strip along a second folding part near a boundary portion with the correction tool body portion at an angle of 180° or larger with respect to the tongue-shaped strip between the first folding part and the second folding part, and in this state subjecting the ingrown nail correction tool to shape-memory heat treatment at 800 to 950°C for 1 to 60 minutes and aging treatment at an aging temperature of 100 to 200°C for 10 to 60 minutes.

## Amended claims

### Amended claims under Art. 19.1 PCT

**1.** Amended) An ingrown nail correction tool made of a super-elastic alloy for correcting an ingrown nail, comprising:
a plate-like shape correction tool body portion; and
a plurality of nail holder portions formed at side portions of the correction tool body portion, wherein:
the ingrown nail correction tool is made of a material prepared by subjecting a cold rolled plate material of a Cu-Al-Mn based super-elastic alloy to shape-memory heat treatment;
the ingrown nail correction tool is formed such that a longitudinal direction of the correction tool body portion is substantially perpendicular to a rolling direction of the cold rolled plate material and the nail holder portions are capable of holding a front edge of an ingrown nail that is a subject of correction with the longitudinal direction of the correction tool body portion being a width direction of the ingrown nail, whereby a correction force of the correction tool body portion for correcting the ingrown nail is better than a case in which the ingrown nail correction tool is formed such that the longitudinal direction of the correction tool body portion substantially corresponds to the rolling direction of the cold rolled plate material; and
the ingrown nail correction tool is formed by folding such that a direction in which the nail holder portions are formed substantially corresponds to the rolling direction of the cold rolled plate material, whereby the nail holder portions have better durability than a case in which the nail holder portions are formed substantially perpendicularly to the rolling direction of the cold rolled plate material.

**2.** Amended) The ingrown nail correction tool of Claim 1, wherein:
the nail holder portions are formed by folding along a first folding part near a front end portion of the nail holder portions a tongue-shaped strip formed substantially perpendicularly to the longitudinal direction of the correction tool body portion, and also folding the tongue-shaped strip along a second folding part near a boundary portion with the correction tool body portion;
at the second folding part, the tongue-shaped strip is folded at an angle of 180° or larger with respect to a direction in which the tongue-shaped strip is formed; and
at the first folding part, the tongue-shaped strip is folded at an angle larger than 180° with respect to the nail holder portions between the first folding part and the second folding part.

**3.** Amended) The ingrown nail correction tool of Claim 1, wherein the Cu-Al-Mn based super-elastic alloy which is a material of the ingrown nail correction tool is an alloy comprising 3 to 10 wt% of Al, 5 to 20 wt% of Mn, Cu as the balance and an inevitable impurity.

**4.** Amended) The ingrown nail correction tool of Claim 1, wherein the Cu-Al-Mn based super-elastic alloy which is a material of the ingrown nail correction tool is an alloy comprising 3 to 10 wt% of Al, 5 to 20 wt% of Mn, 0.001 to 10 wt% of one or more of Ni, Co, Fe, Ti, V, Cr, Si, Nb, Mo, W, Sn, Sb, Mg, P, Be, Zr, Zn, B, C, Ag, and misch metal, Cu as the balance and an inevitable impurity.

**5.** Cancelled)

**6.** The ingrown nail correction tool of Claim 1, wherein the correction tool body portion is curved in a planar direction of the correction tool body portion that is substantially perpendicular to the direction in which the nail holder portions are formed.

**7.** The ingrown nail correction tool of Claim 1, wherein the ingrown nail correction tool is surface treated to improve corrosion resistance.

**8.** Cancelled)

**9.** Cancelled)

**10.** New) A method of improving a correction force and durability of an ingrown nail correction tool for correcting an ingrown nail that comprises a plate-like shape correction tool body portion having and a plurality of nail holder portions formed at side portions of the correction tool body portion and being capable of holding a front end of an ingrown nail and is made of a material prepared by subjecting a cold processed plate material of a Cu-Al-Mn based super-elastic alloy to shape-memory heat treatment, wherein:
the nail holder portions are formed by folding a tongue-shaped strip along a direction substantially perpendicular to a longitudinal direction of the correction tool body portion;
the ingrown nail correction tool is arranged such that the longitudinal direction of the correction tool body portion is substantially perpendicular to a rolling direction of the cold rolled plate material and the longitudinal direction of the correction tool body portion corresponds to a width direction of an ingrown nail that is subject to correction, whereby the correction force of the ingrown nail correction tool is improved; and
the nail holder portions are formed by folding such that the direction in which the nail holder portions are formed substantially corresponds to the rolling direction of the cold rolled plate material, whereby the durability of the nail holder portions is improved.

**11.** New) The method of Claim 10, wherein: the tongue-shaped strip is folded along a first folding part near a front end portion and folded along a second folding part near a boundary portion with the correction tool body portion; at the second folding part, the tongue-shaped strip is folded at an angle of 180° or larger with respect to a direction in which the tongue-shaped strip is formed; and at the first folding part, the tongue-shaped strip is folded at an angle larger than 180° with respect to the nail holder portions between the first folding part and the second folding part.

Statement under Art. 19.1 PCT
Claim 1 has been amended based on the paragraphs [0039], [0046], [0054], [0059] and [0062] of the description to incorporate the technical features of previous Claims 1 and 2 and clarify the effect produced by arranging such that the longitudinal direction of the correction tool body is substantially perpendicular to the rolling direction of the cold rolled plate material and the effect produced by arranging such that the direction in which the nail holder portions are formed substantially corresponds to the rolling direction of the cold rolled plate material.

Claim 2 incorporates the technical feature of previous Claim 5.

Claims 3 and 4 have been amended to amend the claim number from which they depend, in response to the amendment of Claim 1.

Previous Claims 5, 8 and 9 have been cancelled.

Claim 10 has been newly added and is directed to an invention relating to a method of improving a corrective force and durability of the ingrown nail correction tool, which is supported by the paragraphs [0016], [0039], [0046], [0054], [0059] and [0062] of the description. Claim 10 incorporates a technical feature corresponding to Claim 1.

Claim 11 depends from Claim 10 and is directed to a method invention corresponding to Claim 2.
